# EUROPEAN PATENT APPLICATION

(11) **EP 2 436 351 A1**
(43) Date of publication of application: **04.04.2012**
(21) Application number: 10780494.0
(22) Date of filing: 24.05.2010
(51) Int. Cl.: A61F 13/15, A61F 13/49, A61F 13/514

(54) **ABSORPTIVE ARTICLE MANUFACTURING METHOD AND ABSORPTIVE ARTICLE**

(30) Priority: 25.05.2009 JP 2009125896
(71) Applicant: Unicharm Corporation, Ehime 799-0111 (JP)
(72) Inventor: OKU, Tomomi, Kanonji-shi Kagawa 769-1602 (JP); SAKAGUCHI, Satoru, Kanonji-shi Kagawa 769-1602 (JP); OGASAWARA, Yoshikazu, Kanonji-shi Kagawa 769-1602 (JP); ITO, Noriaki, Kanonji-shi Kagawa 769-1602 (JP)
(74) Representative: Knights, Rupert
(86) International application number: PCT/JP2010/058699
(87) International publication number: WO 2010/137539

(57) **Abstract**

In a method of manufacturing an absorbent article, an absorbent article 1 is manufactured in which backsheets are in continuation, in a state in which the longitudinal direction of the absorbent article 1 corresponds to the conveyance direction MD of a continuous body 120. The method the absorbent article includes: a step of pressing the continuous body 120 in a thickness direction T of the continuous body 120 by a pair of embossing roll mechanisms 520, a step of expanding the continuous body 120 that has been pressed towards outside a widthwise direction CD of the continuous body 120 until it becomes flat, by a pair of expansion roll mechanisms 600, and a step of joining a absorber 30 with the continuous body 120 of the backsheet.

## Description

### [Technical Field]

The present invention relates to the method of manufacturing an absorbent article in which an absorbent article is manufactured by conveying a longitudinal continuous body in which liquid-impermeable sheets not allowing a liquid to pass through are in continuation, in a state in which the longitudinal direction of the absorbent article corresponds to the conveyance direction of the continuous body and relates also to an absorbent article.

### [Background Art]

Conventionally in an absorbent article such as a pant-type diaper, a liquid-impermeable sheet formed from polyethylene or the like is provided. Generally a liquid-impermeable sheet is particularly hard even among the sheets that configure an absorbent article. Therefore, as compared to a soft sheet, the liquid-impermeable sheet does not easily follow the movement of the wearer, and causes a decline in comfort when the absorbent article is worn.

Thus, a technique of softening the liquid-impermeable sheets by reducing the thickness of the longitudinal continuous body having liquid-impermeable sheets in continuation with the use of an embossing roll mechanism is known (for example, see Patent Document 1).

The embossing roll mechanism includes a first embossing roll having a plurality of zigzag shaped first convex units along a roll axial direction on one surface of the continuous body and a second embossing roll arranged on the opposite side of the first embossing roll and sandwiching the continuous body and having a plurality of zigzag shaped second convex units along a roll axial direction.

The continuous body is passed between the first embossing roll and the second embossing roll in a state where it is pulled to the conveyance direction of the continuous body by the conveyor configured to convey the continuous body At this point, by fitting the plurality of second convex units between the plurality of first convex units so as to sandwich the continuous body the continuous body is pressed (embossed) in a thickness direction. Thus, the continuous body is extended in the thickness direction and is softened.

However, the aforementioned conventional method of manufacturing an absorbent article had the following problems. That is, irregularities are formed in the thickness direction in the liquid-impermeable sheet formed from the continuous body. Therefore, the problem was that the liquid-impermeable sheet in which irregularities were formed would touch the wearer, and would cause a feeling of discomfort to the wearer.

For example, if the liquid-impermeable sheet were used as the outermost sheet (the so-called backsheet) from the wearer, it would form an outline of the absorbent article thereby deteriorating the texture of the absorbent article.

### [Related Art Document]

### [Patent Document]

[Patent Document 1]Japanese Unexamined Patent Application Publication (Translation of PCT Application) No. 2001-509420 (pages 16 to 18, Fig. 3)

### [Summary of Invention]

The first feature of the present invention is summarized in that a method of manufacturing an absorbent article in which an absorbent article is manufactured by conveying a longitudinal continuous body in which liquid-impermeable sheets not allowing a liquid to pass through are in continuation, in a state in which the longitudinal direction of the absorbent article corresponds to the conveyance direction of the continuous body the method including: a step of pressing the flat continuous body in a thickness direction of the continuous body by a pair of embossing roll mechanisms in which a plurality of convex units are formed on a circumference, and in between the convex units of one side, the convex units of the other side are fitted so as to sandwich the continuous body, a step of expanding the continuous body that has been pressed towards outside a widthwise direction of the continuous body until it becomes flat, by a pair of expansion roll mechanisms provided at the side of both side units in the widthwise direction of the continuous body and a step of joining a component member configuring the absorbent article with the expanded continuous body wherein each of the expansion roll mechanisms has inner ends (inner ends 612A) positioned inside the widthwise direction of the continuous body and outer ends (outer ends 612B) positioned outside the widthwise direction of the continuous body and includes a pair of pressing rolls (first upper pressing roll 611, first lower pressing roll 612, second upper pressing roll 621 and second lower pressing roll 622)that sandwich the continuous body and each of the inner ends is positioned at the tip of the conveyance direction of the continuous body from the outer ends.

### [Brief Description of Drawings]

[Fig. 1] Fig. 1 is a plan view showing an absorbent article 1 according to the present embodiment.
[Fig. 2] Fig. 2 is a cross-sectional view (cross-sectional view of A-A of Fig. 1) showing the absorbent article 1 according to the present embodiment.
[Fig. 3] Fig. 3 is a cross-sectional view (cross-sectional view of B-B of Fig. 1) showing the absorbent article 1 according to the present embodiment.
[Fig. 4] Fig. 4 is a plan view/magnified cross-sectional view (part 1) showing a backsheet 20 according to the present embodiment.
[Fig. 5] Fig. 5 is a plan view (part 2) showing the backsheet 20 according to the present embodiment.
[Fig. 6] Fig. 6 is a diagram for explaining the method of manufacturing the absorbent article according to the present embodiment.
[Fig. 7] Fig. 7 is a perspective view showing an embossing device 500 according to the present embodiment.
[Fig. 8] Fig. 8 is a side view (fragmentary view of A of Fig. 7) showing the embossing device 500 according to the present embodiment.
[Fig. 9] Fig. 9 is a front view showing the embossing device 500 according to the present embodiment.
[Fig. 10] Fig. 10 is a magnified front view showing the embossing device 500 according to the present embodiment.
[Fig. 11] Fig. 11 is a perspective view showing an expansion roll mechanism 600 according to the present embodiment.
[Fig. 12] Fig. 12 is a top view (fragmentary view of A of Fig. 11) showing the expansion roll mechanism 600 according to the present embodiment.

### [Description of Embodiments]

Hereinafter, a method of manufacturing an absorbent article and an absorbent article according to an embodiment is described with reference to drawings. In the following description of the drawings, the same or similar reference numerals are used to designate the same or similar parts. It will be appreciated that the drawings are schematically shown and the ratio and the like of each dimension are different from the real ones. Therefore, detailed dimension and the like should be determined considering the following description. Of course, among the drawings, the dimensional relationship and the ratio are different.

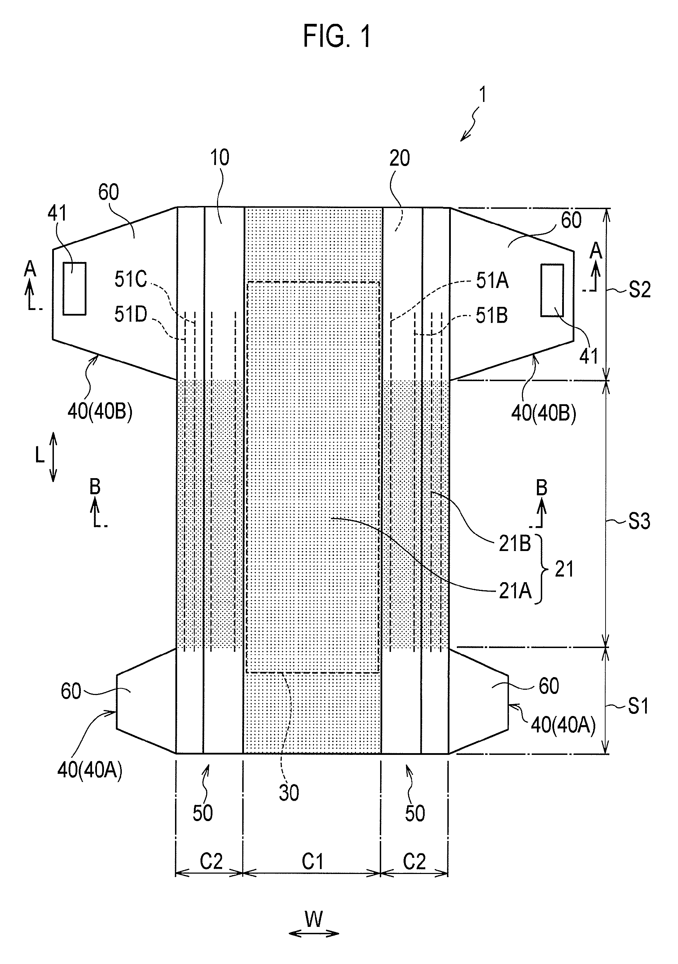

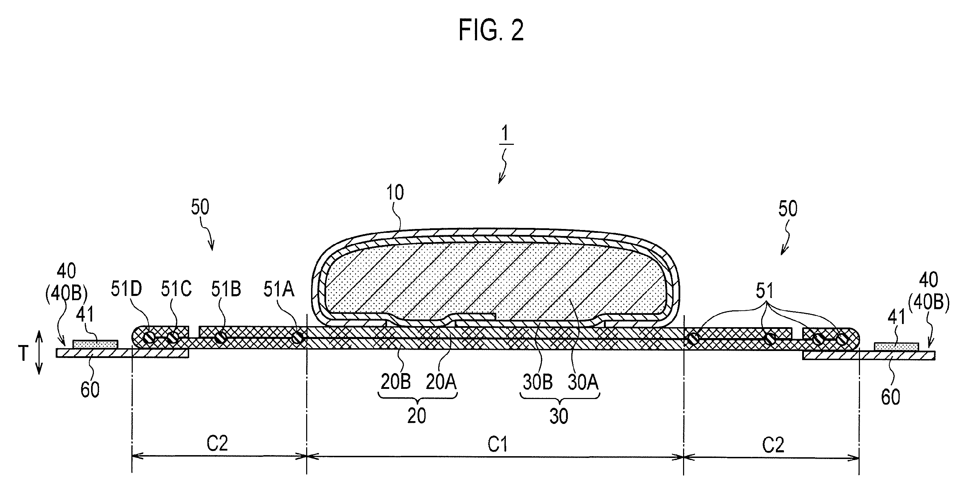

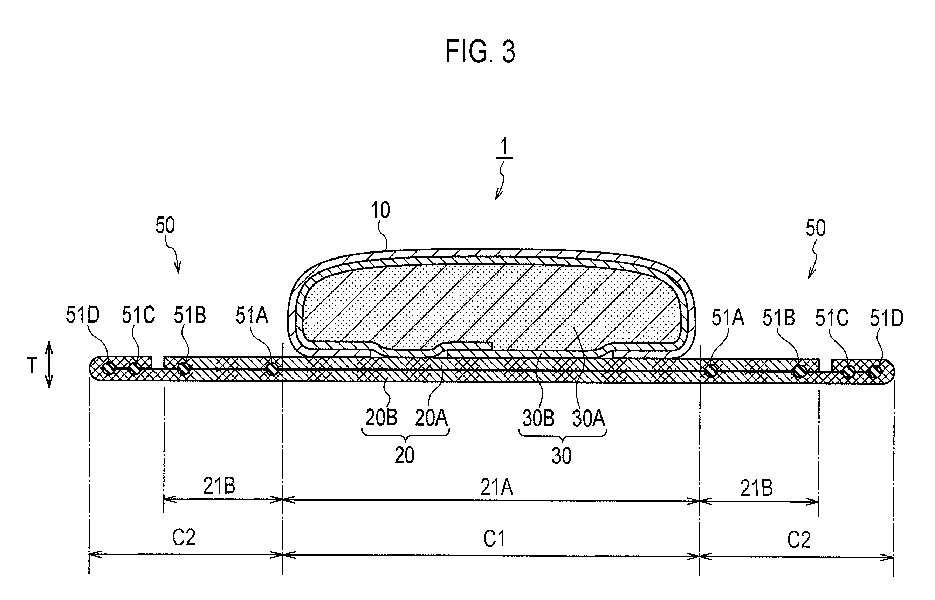

First of all, a configuration of an absorbent article 1 according to the present embodiment is explained with reference to drawings. Fig. 1 is a plan view showing the absorbent article 1 according to the present embodiment. Fig. 2 is a cross-sectional view (cross-sectional view of A-A of Fig. 1) showing the absorbent article 1 according to the present embodiment. Fig. 3 is a cross-sectional view (cross-sectional view of B-B of Fig. 1) showing the absorbent article 1 according to the present embodiment. Note that in the present embodiment, the absorbent article 1 is an open-type diaper.

As shown in Fig. 1 through Fig. 3, the absorbent article 1 has a longitudinally elongated shape towards the front-back direction (hereinafter, the longitudinal direction L of the absorbent article 1) facing the back waistline (back side) from the front waistline (stomach side) of the wearer.

The absorbent article 1 has a front waistline region S1 corresponding to the front waistline of the wearer, a back waistline region S2 corresponding to the back waistline of the wearer, and a crotch region S3 corresponding to the crotch of the wearer and positioned between the front waistline region S1 and the back waistline region S2, in the longitudinal direction L of the absorbent article 1.

Furthermore, the absorbent article 1 has a central region C1 including an absorber 30 (described later), and a pair of side regions C2 positioned outside the central region C1 with respect to a widthwise direction W of the absorbent article 1, in the widthwise direction W perpendicular to the longitudinal direction L of the absorbent article 1.

Such an absorbent article 1 includes a topsheet 10, a backsheet 20 (liquid-impermeable sheet), and an absorber 30. Furthermore, a waist flap unit 40 and a side flap unit 50 are provided in the absorbent article 1.

The topsheet 10 is provided on the side that is in contact with the skin of the wearer. The topsheet 10 is arranged so as to wrap around the absorber 30. The topsheet 10 is formed by a liquid-permeable sheet, such as a hydrophilic nonwoven cloth and woven cloth, an aperture plastic film, or an aperture hydrophobic nonwoven cloth.

The backsheet 20 is provided outside the absorbent article 1 at the time of wearing. The backsheet 20 is formed from a liquid-impermeable back sheet 20A such as a water-resistive film (for example, polyethylene) and an exterior sheet 20B such as a nonwoven cloth pasted on a surface on the opposite side where the absorber 30 of the back sheet 20A is provided. The backsheet 20 need not necessarily be formed from the back sheet 20A and the exterior sheet 20B, and may comprise only back sheet 20A. The backsheet 20 is described later in detail.

The absorber 30 is provided between the topsheet 10 and the backsheet 20. The absorber 30 absorbs the bodily fluid of the wearer. The absorber 30 is formed from a mixed powder 30A of ground pulp and high absorbent polymer, and a covering material 30B, such as a tissue, for covering the mixed powder 30A.

Here, the aforementioned topsheet 10, backsheet 20, and absorber 30 are each joined by an adhesive (for example, hot melt adhesive) and thermal fusion bonding. Note that the absorber 30 is joined with the backsheet 20 when the stretched unit 21 of the backsheet 20 (described later) is in a flat state.

The waist flap unit 40 is provided in the front waistline region S1 and the back waistline region S2 in the longitudinal direction L of the absorbent article 1. The waist flap unit 40 has a pair of foreside flap units 40A positioned in the front waistline region S1 and a pair of backside flap units 40B positioned in the back waistline region S2.

The foreside flap units 40A are formed when a sidesheet 60 pasted to the backsheet 20 extends towards outside the widthwise direction W of the absorbent article 1. Note that the foreside flap units 40A can also be formed when rather than the sidesheet 60, the backsheet 20 (back sheet 20A and exterior sheet 20B) extends out.

Similar to the foreside flap units 40A, the backside flap units 40B are formed when the sidesheet 60 extends towards outside the widthwise direction W of the absorbent article 1. In the surface on the side where the absorber 30 of the backside flap units 40B is provided, a locking unit 41 configured to lock to the front waistline region S1 is provided outside the widthwise direction W of the absorbent article 1.

The locking unit 41 is formed from a hook-and-loop fastener, for example. In such a case, the hook-and-loop fastener becomes the male member, and a female member is provided as a locked unit in the region where the locking unit 41 of the front waistline region S1 is locked. Note that if the front waistline region S1 is configured from a nonwoven cloth, the front waistline region S1 itself may execute the role of the locked unit.

The side flap unit 50 is provided along the longitudinal direction L of the absorbent article 1 outside the absorber 30 with respect to the widthwise direction W of the absorbent article 1. In the side flap unit 50, a strand 51 formed from a rubber (for example, polyurethane) having elasticity is provided in the longitudinal direction L of the absorbent article 1. The strand 51 is joined with the backsheet 20 in an extended state in the longitudinal direction L of the absorbent article 1.

The strand 51 is configured from a first strand 51A, a second strand 51B, a third strand 51C, and a fourth strand 51D from inside the widthwise direction W of the absorbent article 1 towards the outside. The first strand 51A and the second strand 51B are provided between the back sheet 20A and the exterior sheet 20B. The third strand 51C and the fourth strand 51D are sandwiched by the exterior sheet 20B due to folding back of the exterior sheet 20B extending outside the widthwise direction W of the absorbent article 1 from the back sheet 20A.

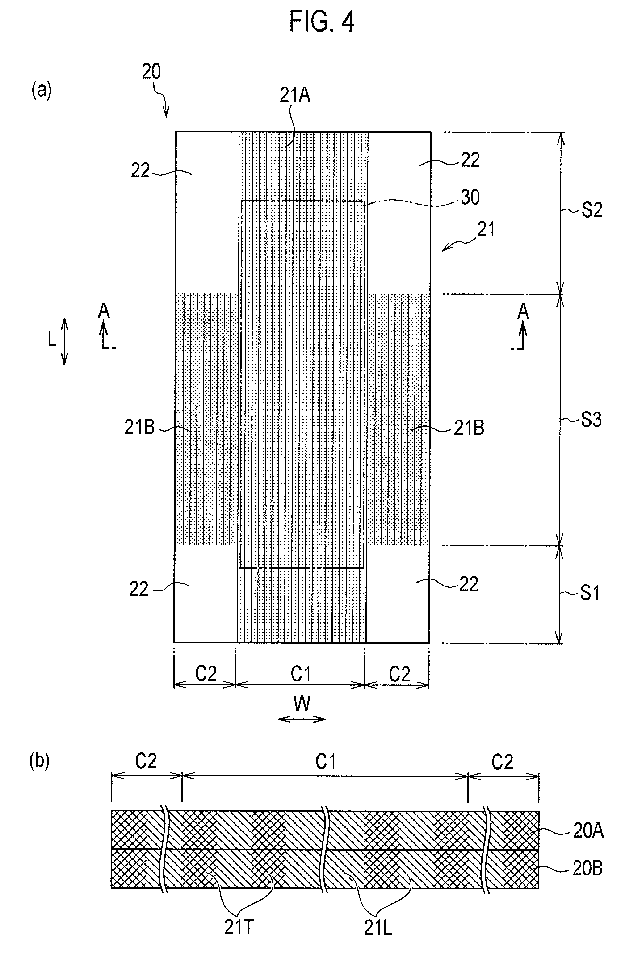

Next, a configuration of the aforementioned backsheet 20 is explained with reference to drawings. Fig. 4 (a) is a plan view showing the backsheet 20 according to the present embodiment, and Fig. 4 (b) is a magnified cross-sectional view (cross-sectional view of A-A of Fig. 4(a)) showing the backsheet 20 according to the present embodiment.

As shown in Fig. 4 (a), the backsheet 20 (back sheet 20A and exterior sheet 20B) has a stretched unit 21 and an un-stretched unit 22.

The stretched unit 21 is a region formed by pressing the backsheet 20 in the thickness direction T of the backsheet 20. That is, the stretched unit 21 is the region with a basis weight (mass per unit area) lesser than that of the un-stretched unit 22 due to the execution of an expansion process (stretching process) described later.

In the stretched unit 21 are provided irregularities in continuation with the longitudinal direction L of the absorbent article 1 and arranged side by side in plurality in the widthwise direction W of the absorbent article 1. Specifically, in the stretched unit 21, as shown in Fig. 4 (b), rough units 21L with a large amount of expansion and dense units 21T whose amount of expansion is lesser than that of the rough units 21L are formed alternately. Thus, minute irregularities are formed in the backsheet 20, and when the irregularities become smooth, the stretched unit 21 stretches in the widthwise direction W of the absorbent article 1.

The stretched unit 21 is desired to be expanded by 2.5 times or less with respect to the backsheet 20 prior to the execution of the expansion process in the backsheet 20. The stretched unit 21 has a central stretched portion 21A positioned in the central region C1 within the crotch region S3, and side stretched portions 21B positioned in the side regions C2 within the crotch region S3. That is, the stretched unit 21 is provided in the entire crotch region S3 in the widthwise direction W of the absorbent article 1. Furthermore, the stretched unit 21 is provided in the entire central region C1 in the longitudinal direction L of the absorbent article 1.

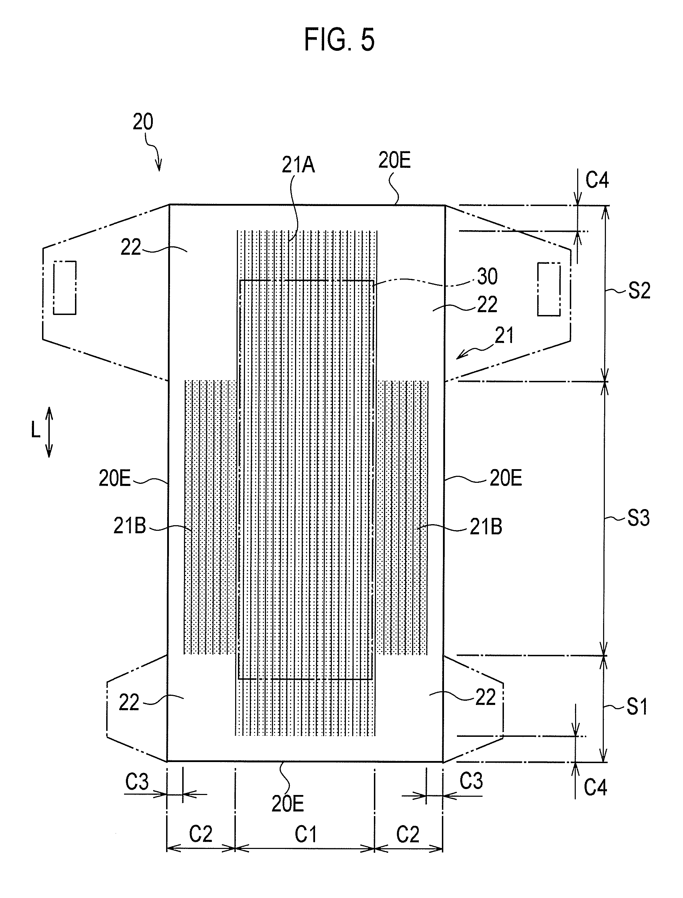

Note that the side stretched portions 21B need not necessarily be provided in the entire crotch region S3, and for example, as shown in Fig. 5, may be provided in the crotch region S3 excluding the edge regions C3 including the edges 20E positioned outside the widthwise direction W of the backsheet 20, and may be provided in at least a part of the crotch region S3. Similarly the central stretched portion 21A need not necessarily be provided in the entire central region C1, and for example, as shown in Fig. 5, may be provided in the central region C1 excluding the edge regions C4 including the edges 20E positioned outside the longitudinal direction L of the absorbent article 1, and may be provided in at least a part of the central region C1.

The un-stretched unit 22 is a region in which the backsheet 20 is not pressed in the thickness direction T of the backsheet 20. That is, the un-stretched unit 22 is the region excluding the stretched unit 21. Unlike the stretched unit 21, the un-stretched unit 22 is not stretched in the widthwise direction W of the absorbent article 1.

The un-stretched unit 22 is provided in at least a part of the front waistline region S1 and the back waistline region S2. That is, the un-stretched unit 22 is provided in the pair of side regions C2 in the front waistline region S1, and in the pair of side regions C2 in the back waistline region S2.

Thus, the aforementioned stretched unit 21 is formed in at least a part of the crotch region S3 in the absorbent article 1. Furthermore, the stretched unit 21 is joined with the absorber 30 in a flat state (see Fig. 2 and Fig. 3).

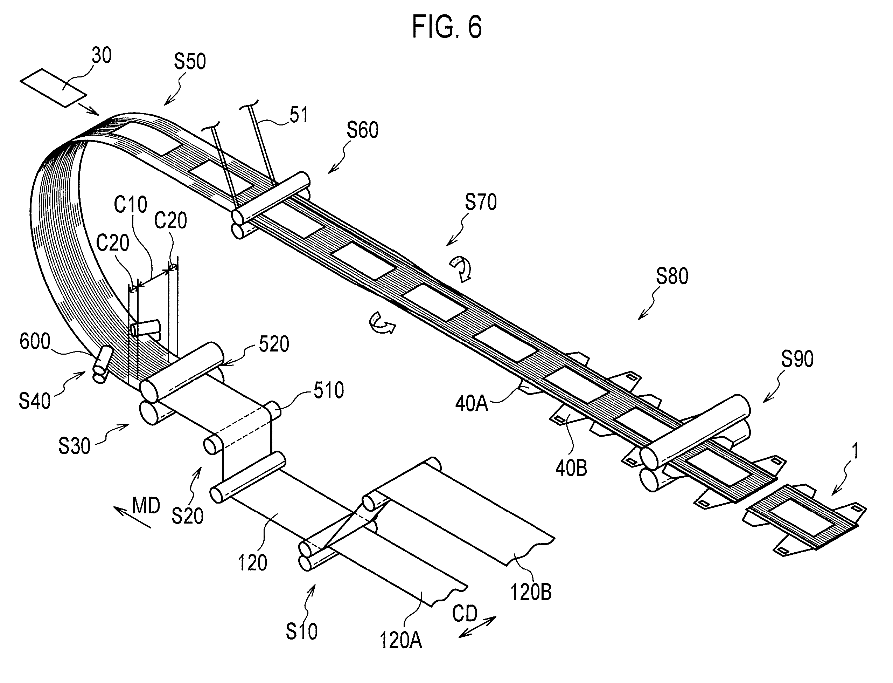

Next, a method of manufacturing an absorbent article for manufacturing the aforementioned absorbent article 1 is explained with reference to drawings. Fig. 6 is a diagram for explaining the method of manufacturing the absorbent article according to the present embodiment.

In the method of manufacturing the absorbent article, the absorbent article 1 is manufactured by conveying a longitudinal backsheet continuous body 120 in which backsheets 20 are in continuation in a state in which the longitudinal direction L of the absorbent article 1 corresponds to the conveyance direction MD of the backsheet continuous body 120 (a so-called vertical flow method).

As shown in Fig. 6, the method of manufacturing the absorbent article includes a sheet-laminating step S10, a sheet-preheating step S20, a sheet-pressing step S30, a sheet-expansion step S40, an absorber-joining step S50, a strand-disposing step S60, a side flap forming step S70, a waist flap disposing step S80, and a product-cutting step S90.

In the sheet-laminating step S10, a longitudinal exterior-sheet continuous body 120B in which exterior sheets 20B are in continuation is laminated to a longitudinal back sheet continuous body 120A in which back sheets 20A are in continuation, and a longitudinal backsheet continuous body 120 in which backsheets 20 are in continuation is formed. Note that the present embodiment is not limited to the pasting of the exterior-sheet continuous body 120B to the back sheet continuous body 120A, and the back sheet 20A may be laminated intermittently to the exterior-sheet continuous body 120B.

In the sheet-preheating step S20, the backsheet continuous body 120 is heated at a predetermined temperature (for example, 100°C) by a preheating roll 510 of the embossing device 500 described later.

In the sheet-pressing step S30, a flat backsheet continuous body 120 is pressed in the thickness direction T of the backsheet continuous body 120 by an embossing roll mechanism 520 in the embossing device 500 described later. Specifically a process (expansion process) of forming irregularities in continuation with the conveyance direction MD of the backsheet continuous body 120 and arranged side by side in plurality in the widthwise direction CD of the backsheet continuous body 120 is executed in the flat backsheet continuous body 120.

At this point, the stretched unit 21 (the central stretched portion 21A and the side stretched portions 21B) is formed in the central region C10 and the side regions C20 of the backsheet continuous body 120. As a result, the pair of side regions C2 in the front waistline region S1, and the pair of side regions C2 in the back waistline region S2 of the absorbent article 1 form the un-stretched unit 22.

Note that the central region C10 of the backsheet continuous body 120 corresponds to the central region C1 of the absorbent article 1 and indicates the region positioned in the center of the widthwise direction CD. The side regions C20 of the backsheet continuous body 120 correspond to the side regions C2 within the crotch region S3 of the absorbent article 1, and indicate the regions outside the widthwise direction CD of the backsheet continuous body 120 from the central region C10.

In the sheet-expansion step S40, the backsheet continuous body 120 is expanded (widened) until it becomes flat towards outside the widthwise direction CD of the backsheet continuous body 120 by the expansion roll mechanism 600 described later.

In the absorber-joining step S50, the absorber 30 in which the mixed powder 30A is covered with the covering material 30B is joined (mounted) on the backsheet continuous body 120 that has become flat by expansion.

In the strand-disposing step S60, the strand 51 is disposed outside the absorber 30 in the widthwise direction CD of the backsheet continuous body 120 in a state when the strand 51 is extended in the conveyance direction MD of the backsheet continuous body 120 on the backsheet continuous body 120 in which the absorber 30 is disposed.

In the side flap forming step S70, by folding back the exterior-sheet continuous body 120B extended out in the widthwise direction CD of the backsheet continuous body 120 from the back sheet continuous body 120A, the strand 51 is sandwiched by the exterior-sheet continuous body 120B.

In the waist flap disposing step S80, the foreside flap units 40A formed beforehand, and the backside flap units 40B on which the locking unit 41 is mounted are disposed outside the absorber 30 in the widthwise direction CD of the backsheet continuous body 120. Thus, the waist flap unit 40 (foreside flap unit 40A and backside flap unit 40B) is formed.

In the product-cutting step S90, the backsheet continuous body 120 in which the absorber 30, the side flap unit 50, and the waist flap unit 40 are disposed is cut in the size of a single product along the widthwise direction CD. Thus, the absorbent article 1 is manufactured.

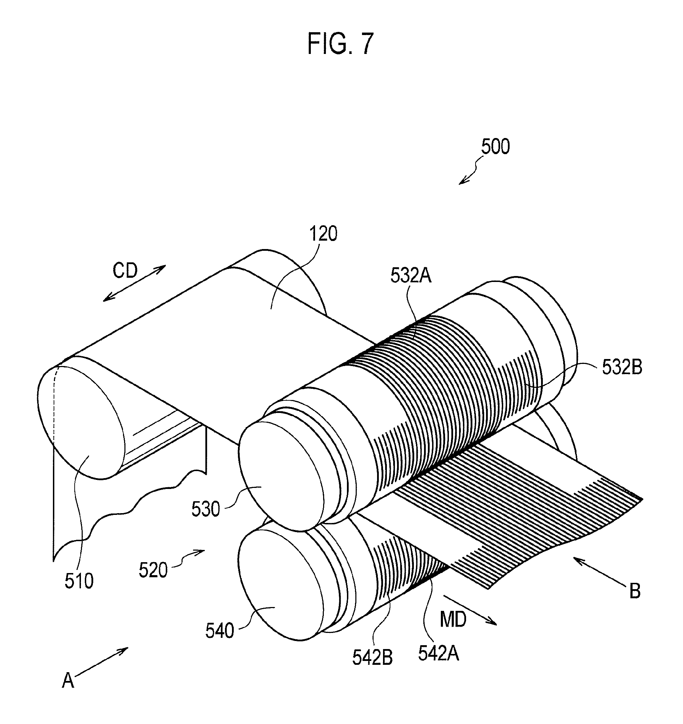

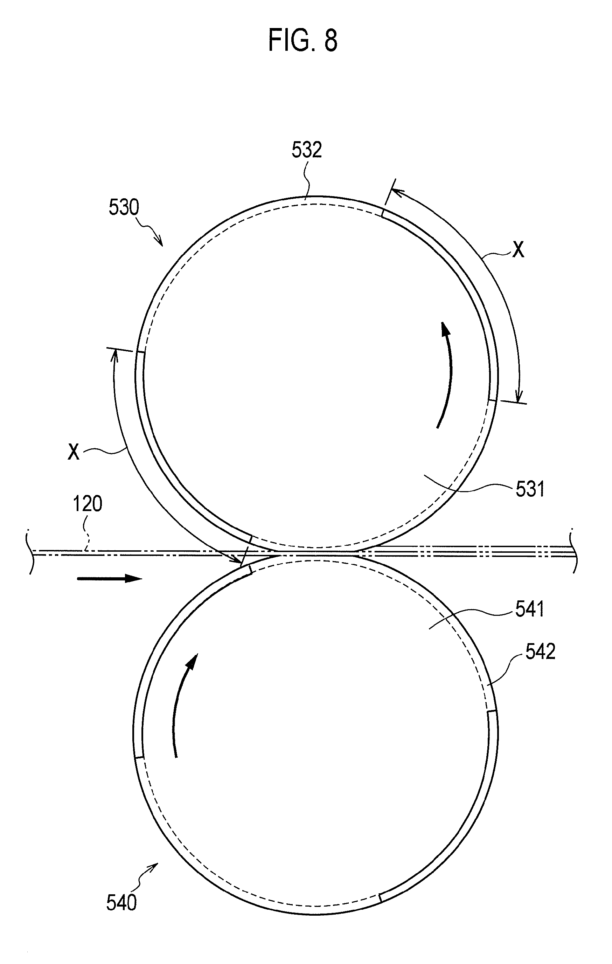

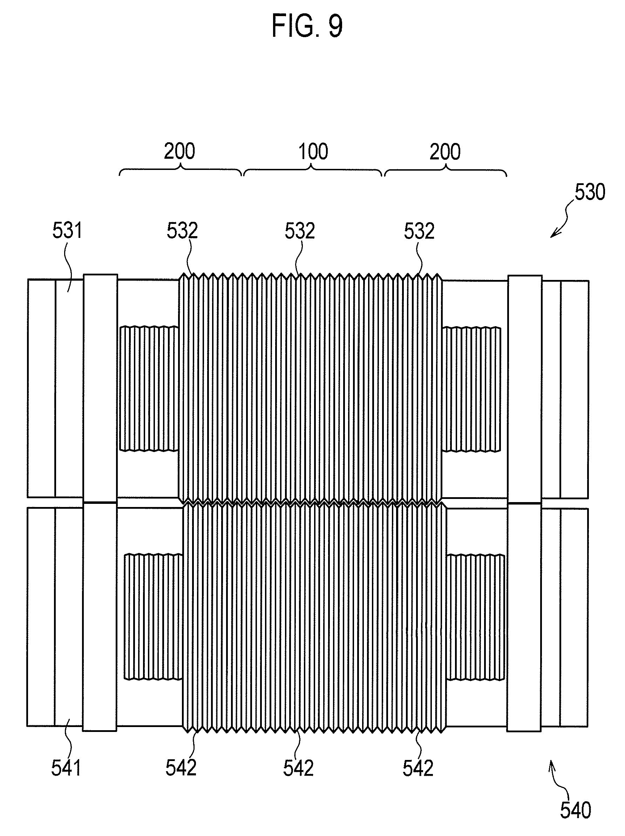

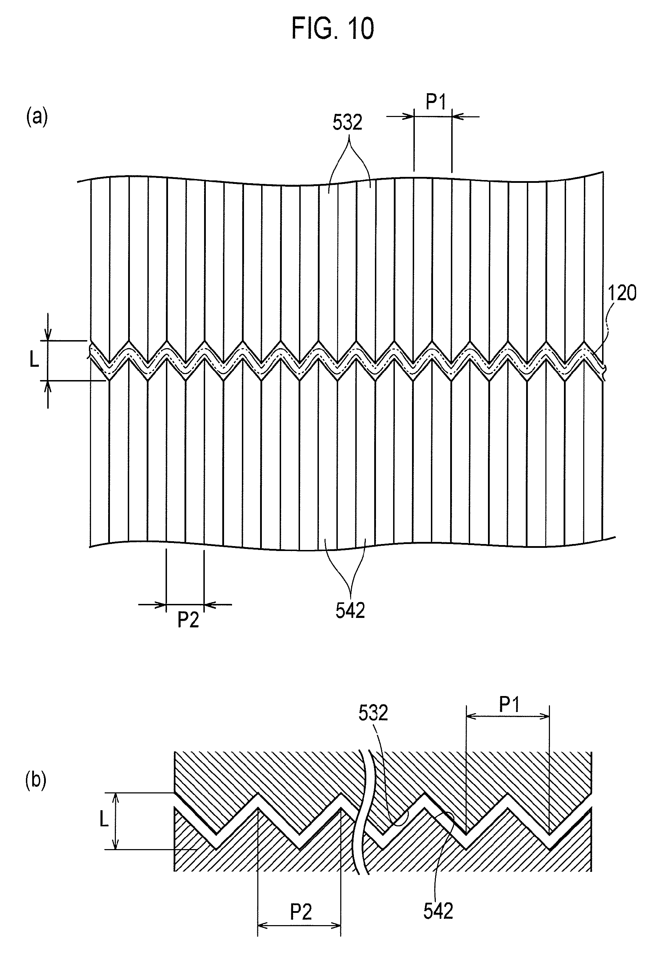

Next, a configuration of the embossing device 500 used in the aforementioned sheet-preheating step S20 and the sheet-pressing step S30 is explained with reference to drawings. Fig. 7 is a perspective view showing the embossing device 500 according to the present embodiment. Fig. 8 is a side view (fragmentary view of A of Fig. 7) showing the embossing device 500 according to the present embodiment. Fig. 9 is a front view (fragmentary view of B of Fig. 7) showing the embossing device 500 according to the present embodiment. Fig. 10 is a magnified front view showing the embossing device 500 according to the present embodiment.

As shown in Fig. 7 through Fig. 10, the embossing device 500 executes the process (expansion process) of pressing the flat backsheet continuous body 120 in the thickness direction T of the backsheet continuous body 120, and then expanding it in the widthwise direction CD of the backsheet continuous body 120. The embossing device 500 includes a preheating roll 510 and an embossing roll mechanism 520.

The preheating roll 510 is provided upstream of the conveyance direction MD of the backsheet continuous body 120 from the embossing roll mechanism 520. Before passing through the embossing roll mechanism 520, the preheating roll 510 heats the backsheet continuous body 120. The preheating roll 510 is set to a predetermined temperature (for example, 100°C). The preheating roll 510 conveys a flat backsheet continuous body 120 in the embossing roll mechanism 520.

The embossing roll mechanism 520 heats the backsheet continuous body 120 that has passed through the preheating roll 510, and at the same time, executes the expansion process in the backsheet continuous body 120. For example, the embossing roll mechanism 520 executes an expansion process by 2.5 times or less with respect to the backsheet continuous body 120 prior to the execution of the expansion process in the backsheet continuous body 120. The embossing roll mechanism 520 has a predetermined temperature (for example, 50 to 80°C).

Such an embossing roll mechanism 520 is configured from a pair of embossing roll mechanisms in which a plurality of convex units are formed on the circumference, and in between the convex units of one side, the convex units of the other side are fitted so as to sandwich the backsheet continuous body 120. Specifically, the embossing roll mechanism 520 includes an upper embossing roll 530 and a lower embossing roll 540.

The upper embossing roll 530 is arranged on one surface (upper surface) of the backsheet continuous body 120. The upper embossing roll 530 has an upper roll main body 531 and a plurality of upper convex units 532.

The upper roll main body 531 rotates around an axial core. The upper convex units 532 press (emboss) the backsheet continuous body 120 on the upper surface of the backsheet continuous body 120. The upper convex units 532 are arranged side by side in plurality in the axial core direction of the upper roll main body 531.

The upper convex units 532 are arranged along the rotation direction of the upper roll main body 531, and are protruding out from the circumference of the upper roll main body 531. The upper convex units 532 take a tapered form (almost a rectangular form) towards the outside from the circumference of the upper roll main body 531 in a cross section in the axial core direction of the upper roll main body 531.

The upper convex units 532 have a plurality of central convex units 532A and a plurality of sideward convex units 532B.

The central convex units 532A form a central stretched portion 21A by pressing the central region C10 of the backsheet continuous body 120. The central convex units 532A are provided across the entire circumference of the upper roll main body 531 along the circumferential direction of the upper roll main body 531 in the central region C100 of the upper role main body 531 corresponding to the central region C10 of the backsheet continuous body 120.

The sideward convex units 532B form a side stretched portion 21B by pressing the side regions C20 positioned outside the central region C10 in the widthwise direction CD of the backsheet continuous body 120. The sideward convex units 532B are provided intermittently along the circumferential direction of the upper roll main body 531 in the side regions C200 of the upper embossing roll 530 corresponding to the side regions C20 of the backsheet continuous body 120. The circumferential direction length (X) of the sideward convex units 532B corresponds to the length in the longitudinal direction L of the absorbent article 1 of the side stretched portion 21B (that is, the length of the crotch region S3).

The lower embossing roll 540 is arranged on the opposite side (lower surface) of the upper embossing roll 530 while sandwiching the backsheet continuous body 120. The lower embossing roll 540 has a lower roll main body 541 and a plurality of lower convex units 542.

The lower roll main body 541 rotates around an axial core. The lower convex units 542 are fitted between the plurality of upper convex units 532. Same as the upper convex units 532, the lower convex units 542 are arranged side by side in plurality in the axial core direction of the lower roll main body 541.

The lower convex units 542 are arranged along the rotation direction of the lower roll main body 541, and are protruding out from the circumference of the lower roll main body 541. The lower convex units 542 take a tapered form (almost a rectangular form) towards the outside from the circumference of the lower roll main body 541 in a cross section in the axial core direction of the lower roll main body 541.

The lower convex units 542 face the plurality of upper convex units 532 while sandwiching the backsheet continuous body 120, and fit into the plurality of upper convex units 532. The lower convex units 542 have a plurality of central convex units 542A and a plurality of sideward convex units 542B. Note that the configuration of the central convex units 542A and the sideward convex units 542B is the same as that of the upper convex units 532 (central convex units 532A and sideward convex units 532B). Therefore, the explanation of the central convex units 542A and the sideward convex units 542B is omitted.

Thus, due to the fitting of the lower convex units 542 between the plurality of upper convex units 532, the backsheet continuous body 120 is pressed (embossed) in the thickness direction T. As a result, a stretched unit 21 (central stretched portion 21A and side stretched portions 21B) provided with irregularities is formed in the central region C10 of the backsheet continuous body 120 corresponding to the central region C1 of the absorbent article 1, and in the side regions C20 of the backsheet continuous body 120 corresponding to the side regions C2 in the crotch region S3 of the absorbent article 1.

In such an embossing device 500, the preheating roll 510 is set to 100°C, for example. The embossing roll mechanism 520 is set to 80°. The length (L) in which the upper convex units 532 and lower convex units 542 are fitted is 1.6 mm. The interval (p1) of a plurality of upper convex units 532 and the interval (p2) of a plurality of lower convex units 542 is 2.5 mm. Based on this condition, the embossing device 500 can execute an expansion process by 1.3 times with respect to the backsheet continuous body 120 prior to the execution of the expansion process in the backsheet continuous body 120.

Note that in the embossing device 500, by appropriately changing the aforementioned conditions, the magnitude of expansion of the backsheet 20 in the widthwise direction W of the absorbent article 1 (hereinafter, expansion magnitude) can be changed.

Furthermore, the height of the central convex units 532A may be different from the height of the sideward convex units 532B. Similarly, the height of the central convex units 542A may be different from the height of the sideward convex units 542B. Thus, the expansion magnitude of the central stretched portion 21A and the expansion magnitude of the side stretched portions 21B can be changed.

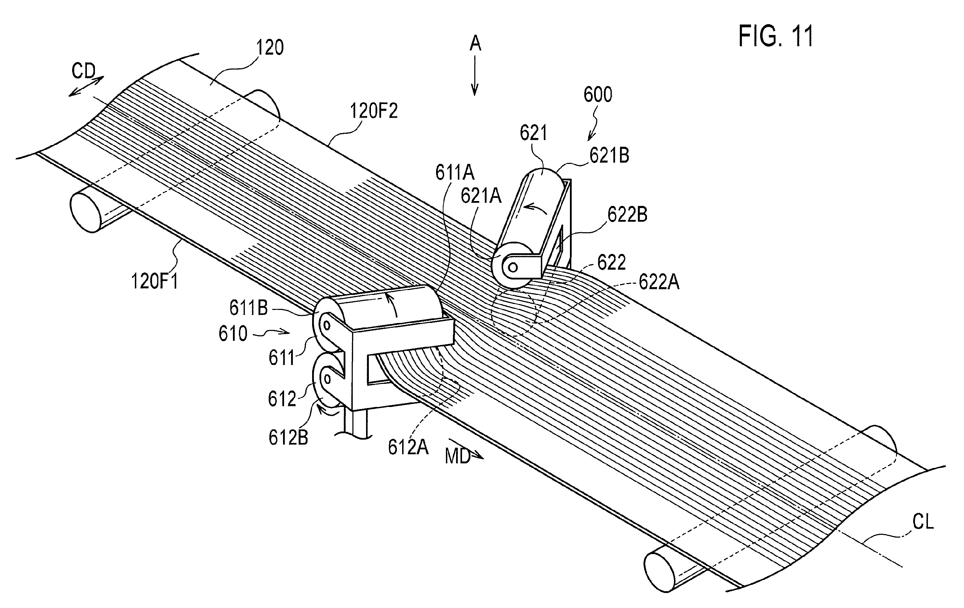

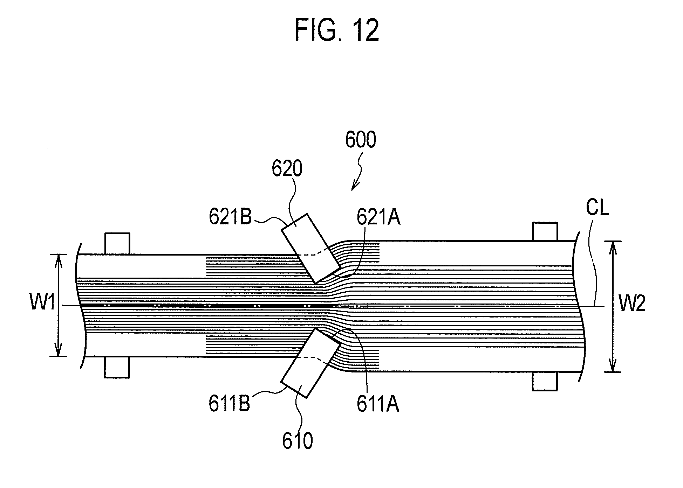

Next, a configuration of the expansion roll mechanism 600 used in the aforementioned sheet expansion step S40 is explained with reference to drawings. Fig. 11 is a perspective view showing an expansion roll mechanism 600 according to the present embodiment. Fig. 12 is a top view (fragmentary view of A of Fig. 11) showing the expansion roll mechanism 600 according to the present embodiment.

As shown in Fig. 11 and Fig. 12, the expansion roll mechanism 600 expands the backsheet continuous body 120 that is pressed by the embossing roll mechanism 520 towards the outside of the widthwise direction CD of the backsheet continuous body 120 until it becomes flat. That is, as shown in Fig. 12, the expansion roll mechanism 600 expands from the width W1 of the backsheet continuous body 120 before it passes through the expansion roll mechanism 600 to the width W2 of the backsheet continuous body 120 after it passes through the expansion roll mechanism 600. As a result, the irregularities provided in the stretched unit 21 become flat, and the stretched unit 21 is expanded (stretched).

Such an expansion roll mechanism 600 is configured from a pair of expansion roll mechanisms provided at the side of the both side units of the widthwise direction CD of the backsheet continuous body 120. Specifically, the expansion roll mechanism 600 includes a first expansion roll mechanism 610 and a second expansion roll mechanism 620.

The first expansion roll mechanism 610 sandwiches one side unit 120E1 (side region C20) of the backsheet continuous body 120. The first expansion roll mechanism 610 includes a pair of pressing rolls. Specifically, it includes the first upper pressing roll 611 and the first lower pressing roll 612.

The first upper pressing roll 611 is arranged on one surface (upper surface) of the backsheet continuous body 120. While being in contact with the upper surface of the backsheet continuous body 120, the first upper pressing roll 611 rotates around the axial core along the delivery direction in which the backsheet continuous body 120 is delivered.

Specifically the first upper pressing roll 611 has an inner end 611A and an outer end 611B. The inner end 611A is positioned inside the widthwise direction W of the backsheet continuous body 120. The outer end 611B is positioned outside the widthwise direction W of the backsheet continuous body 120. The outer end 611B is positioned outside the widthwise direction W of the backsheet continuous body 120 from the side unit 120E 1 of the backsheet continuous body 120.

The first lower pressing roll 612 is arranged on the opposite side (lower surface) of the first upper pressing roll 611 while sandwiching the backsheet continuous body 120. The first lower pressing roll 612 sandwiches the backsheet continuous body 120 in between the first upper pressing roll 611. While being in contact with the lower surface of the backsheet continuous body 120, the first lower pressing roll 612 rotates around the axial core along the delivery direction in which the backsheet continuous body 120 is delivered.

The first lower pressing roll 612 has an inner end 612A and an outer end 612B. The inner end 612A is positioned inside the widthwise direction W of the backsheet continuous body 120. The outer end 612B is positioned outside the widthwise direction W of the backsheet continuous body 120. The outer end 612B is positioned outside the widthwise direction W of the backsheet continuous body 120 from the side unit 120E1 of the backsheet continuous body 120.

Here, the inner end 611A and the inner end 612A are positioned forward from the outer end 611B and the outer end 612B in the conveyance direction MD of the backsheet continuous body 120. That is, in the plan view of the backsheet continuous body 120, the first expansion roll mechanism 610 (first upper pressing roll 611 and the first lower pressing roll 612) is inclined with respect to the widthwise direction CD of the backsheet continuous body 120 to increase the width of the backsheet continuous body 120.

The second expansion roll mechanism 620 sandwiches the other side unit 120E2 (side region C20) of the backsheet continuous body 120. Same as the first expansion roll mechanism 610, the second expansion roll mechanism 620 has a pair of pressing rolls.

Specifically the second expansion roll mechanism 620 includes the second upper pressing roll 621 having an inner end unit 621A and an outer end unit 621B, and the second lower pressing roll 622 having an inner end unit 622A and an outer end unit 622B. Note that the configuration of the second expansion roll mechanism 620 (second upper pressing roll 621 and second lower pressing roll 622) is same as that of the first expansion roll mechanism 610. Therefore, the explanation of the second expansion roll mechanism 620 is omitted.

In such an expansion roll mechanism 600, by appropriately changing the inclination angle of the first upper pressing roll 611 and the first lower pressing roll 612 with respect to the widthwise direction CD of the backsheet continuous body 120, and the inclination angle of the second upper pressing roll 621 and the second lower pressing roll 622 with respect to the widthwise direction CD of the backsheet continuous body 120, the width of the backsheet 20 can be changed.

In the aforementioned present embodiment, a stretched unit 21 provided with minute irregularities is formed in the backsheet continuous body 120 by being pressed by the embossing roll mechanism 520. Next, the backsheet continuous body 120 is expanded until the minute irregularities that have been formed become flat by the expansion roll mechanism 600. Next, with the minute irregularities that have been formed being flat, the backsheet continuous body 120 is joined with the absorber 30.

Because the backsheet continuous body 120 is conveyed in a state where it is pulled to the conveyance direction MD of the backsheet continuous body 120, the phenomenon by which the width of the backsheet continuous body 120 becomes narrow (the so-called neck-in phenomenon) might occur. However, in the present embodiment, as described above, the absorber 30 is joined on the backsheet continuous body 120 that has become flat by being expanded. That is, before the width of the backsheet continuous body 120 that has become flat becomes narrow, the absorber 30 is joined on the backsheet continuous body 120. Therefore, minute irregularities are maintained in a flat state. Therefore, even when the backsheet 20 touches the wearer, the feeling of discomfort to the wearer can certainly be controlled.

Furthermore, due to the fact that the backsheet 20 is in a flat state, the texture of the absorbent article 1 can also be improved. Furthermore, due to expansion, the backsheet 20 becomes thinner. Therefore, it easily follows the movement of the wearer, and the decline in the comfort when the absorbent article 1 is worn can be prevented. Finally, due to the expansion of the backsheet 20, the width of the backsheet 20 can be increased, and the amount of usage of the backsheet 20 can be reduced.

Thus, when the backsheet 20 is softened, the feeling of discomfort to the wearer can certainly be controlled and the deterioration in the comfort when the absorbent article 1 is worn can be prevented while improving the texture, and at the same time, the amount of usage of the backsheet 20 can be reduced.

Furthermore, the expansion roll mechanism 600 includes the first expansion roll mechanism 610 and the second expansion roll mechanism 620. That is, the expansion roll mechanism 600 sandwiches both side units (side regions C20) of the backsheet continuous body 120. Therefore, the backsheet continuous body 120 can easily be expanded uniformly in the widthwise direction CD of the backsheet continuous body 120 until it becomes flat. Therefore, the formation of irregularities in the thickness direction T of the backsheet 20 can certainly be prevented further.

In the present embodiment, the outer end 611B and the outer end 612B are positioned outside the widthwise direction W of the backsheet continuous body 120 from 120E1 of the backsheet continuous body 120. Furthermore, the outer end unit 621B and the outer end unit 622B are positioned outside the widthwise direction W of the backsheet continuous body 120 from 120E2 of the backsheet continuous body 120. Note that if each outer end is inside the side unit of the backsheet continuous body 120, the edges on the side units of the backsheet continuous body 120 cannot be sandwiched, and the backsheet continuous body 120 cannot easily be widened.

In the embodiment, the stretched unit 21 is desired to be expanded by 2.5 times or less with respect to the backsheet 20 prior to the execution of the expansion process in the backsheet 20. Note that if the expansion magnitude of the stretched unit 21 is more than 2.5 times, the backsheet 20 might become too thin, and damages such as breakage and perforation of the backsheet 20 might occur.

Here, if the height of the central convex units 532A is more than the height of the sideward convex units 532B, the expansion magnitude of the central stretched portion 21A becomes more than the expansion magnitude of the side stretched portions 21B. In such a case, because the side regions C2 can be expanded easily as compared with the central region C1, the folding over of the side edges of the backsheet continuous body 120 can be prevented easily in a more certain way in the steps after the execution of the expansion process in the backsheet continuous body 120 (that is, the steps after the sheet-pressing step S30).

On the other hand, if the height of the central convex units 532A is less than the height of the sideward convex units 532B, the expansion magnitude of the central stretched portion 21A becomes less than the expansion magnitude of the side stretched portions 21B. In such a case, because the side regions C2 can be expanded easily as compared with the central region C1, a gap (the so-called pocket) is formed easily between the crotch of the wearer and the absorber 30. Therefore, until the bodily fluid from the wearer collects in the pocket, it can be absorbed by the absorber 30, and even if the bodily fluid crosses over the absorber 30, it can be stopped by the side flap units 50, and side leakage can certainly be prevented.

### (Other embodiments)

So far, the present invention is disclosed through the above embodiment. However, it should not be interpreted that the statements and drawings constituting a part of the present disclosure limit the present invention. From this disclosure, a variety of alternate embodiments, examples, and applicable techniques will be apparent to one skilled in the art.

For example, the embodiment can be changed as follows. Specifically, the explanation is based on the fact that the absorbent article 1 is an open-type diaper, but the embodiment is not limited thereto, and a pant-type diaper and sanitary napkin, or a panty liner can also be used.

Furthermore, the topsheet 10 and backsheet 20, the absorber 30, the waist flap unit 40, and side flap unit 50 are not limited to the configuration explained in the embodiment, and may have a different configuration, and can be changed appropriately according to the purpose. For example, the explanation is based on the fact that the locking unit 41 provided in the backside flap unit 40B is formed from a hook-and-loop fastener, but it is not limited thereto, and also be formed from an adhesive tape.

Furthermore, the backsheet 20 is explained as the liquid-impermeable sheet, but it is not limited thereto, and even a sheet other than the backsheet 20 may be used as a sheet that does not allow the liquid to pass through. Finally the explanation is based on the fact that the strand 51 is rubber, but it is not limited thereto, and for example, a sheet having elasticity or a member having elasticity can be used.

Furthermore, the explanation is based on the fact that the upper embossing roll 530 has upper convex units 532 that protrude out from the upper roll main body 531, but it is not limited thereto, and instead of upper convex units 532, it may have concave units caving in from the upper roll main body 531. Similarly the lower embossing roll 540 need not necessarily have lower convex units 542, and may have concave units caving in from the lower roll main body 541. Note that the configuration of the lower convex units 542 need not necessarily be the same as that of the upper convex units 532, and may be different from that of the upper convex units 532.

Furthermore, the embossing device 500 is explained as an example of forming the stretched unit 21 in the backsheet 20, but it may have any other configuration as long as a stretched unit 21 can be formed in the backsheet 20. For example, the embossing device 500 need not necessarily include the preheating roll 510 and the embossing roll mechanism 520, and may be configured only from the embossing roll mechanism 520. Furthermore, it is obvious that the configuration of the upper convex units 532 and the lower convex units 542 varies depending on the location where the stretched unit 21 is provided.

Furthermore, the explanation is based on the fact that the configuration of the central convex unit 542A and the sideward convex units 542B in the lower embossing roll 540 is the same as that of the upper convex units 532 (central convex unit 532A and sideward convex units 532B), but it is not limited thereto, and the configuration may be slightly different from that of the upper convex units 532 as long as the stretched unit 21 can be formed in the backsheet 20.

Furthermore, the explanation is based on the fact that the expansion roll mechanism 600 includes the first expansion roll mechanism 610 and the second expansion roll mechanism 620, but it is not limited thereto, and may be configured by at least the first expansion roll mechanism 610.

Furthermore, the method of manufacturing an absorbent article is not limited to the explanation of the aforementioned embodiment, and appropriate selection can be made according to the purpose. For example, the explanation is based on the fact that after the sheet-expansion step S40, the absorber-joining step S50 is executed, but it is not limited thereto, and after the sheet-expansion step S40, a component member (such as a sheet) other than the absorber 30 that configures the absorbent article 1 can also be joined.

As described above, it is a matter of course that the present embodiment encompasses a variety of embodiments which have not been described herein. Therefore, technical scope of the present embodiment is defined only by the specific matters of the invention according to the claims that are reasonable from the foregoing description.

The entire contents of Japanese Patent Application No. 2009-125896 (filed on May 25, 2009) are incorporated in the present specification by way of reference.

### [Industrial Applicability]

According to the embodiment, when a longitudinal continuous body in which the liquid-impermeable sheets are in continuation is softened, a method of manufacturing an absorbent article in which the feeling of discomfort to the wearer can certainly be prevented along with an improvement in the texture, as well as an absorbent article can be provided.

## Claims

1. A method of manufacturing an absorbent article in which an absorbent article is manufactured by conveying a longitudinal continuous body in which liquid-impermeable sheets not allowing a liquid to pass through are in continuation, in a state in which the longitudinal direction of the absorbent article corresponds to the conveyance direction of the continuous body the method comprising:
a step of pressing the flat continuous body in a thickness direction of the continuous body by a pair of embossing roll mechanisms in which a plurality of convex units are formed on a circumference, and in between the convex units of one side, the convex units of the other side are fitted so as to sandwich the continuous body a step of expanding the continuous body that has been pressed, towards the outside in a widthwise direction of the continuous body until it becomes flat, by a pair of expansion roll mechanisms provided at the side of both side units in the widthwise direction of the continuous body, and
a step of joining a component member configuring the absorbent article with the expanded continuous body wherein
each of the expansion roll mechanisms has an inner end positioned inside the widthwise direction of the continuous body and an outer end positioned outside the widthwise direction of the continuous body, and includes a pair of pressing rolls that sandwich the continuous body and
each of the inner ends is positioned forward from the outer ends in the conveyance direction of the continuous body

2. The method of manufacturing an absorbent article according to claim 1, wherein the component member is an absorber configured to absorb the bodily fluid of the wearer.

3. The method of manufacturing an absorbent article according to claim 1 or 2, wherein the outer ends are positioned outside from the side units of the continuous body in the widthwise direction of the continuous body.

4. An absorbent article comprising at least a liquid-impermeable sheet not allowing a liquid to pass through, and an absorber configured to absorb the bodily fluid of a wearer, wherein
the liquid-impermeable sheet has a stretched unit provided with irregularities in continuation with the longitudinal direction of the absorbent article and arranged side by side in plurality in the widthwise direction of the absorbent article,
and
in a state when the plurality of irregularities formed in the stretched unit become flat, the liquid-impermeable sheet and the absorber are joined.
